Europäisches Patentamt

**European Patent Office**

**Office européen des brevets**

(11) Publication number: **0 411 521 A1**

## (12) EUROPEAN PATENT APPLICATION

(21) Application number: **90114556.5**

(22) Date of filing: **30.07.90**

(51) Int. Cl.⁵: **A61M 39/00**

(30) Priority: **01.08.89 US 388267**

(43) Date of publication of application:
**06.02.91 Bulletin 91/06**

(84) Designated Contracting States:
**BE DE FR GB IT NL SE**

(71) Applicant: **ABBOTT LABORATORIES**
**CHAD-0377, AP6D/2, One Abbott Park Road**
**Abbott Park, Illinois 60064-3500(US)**

(72) Inventor: **Larkin, Mark E.**
**419 Northgate**
**Lindenhurst Illinois 60046(US)**
Inventor: **Kramer, David E.**
**28 Chestnut Road**
**Northbrook Illinois 60062(US)**

(74) Representative: **Modiano, Guido et al**
**MODIANO, JOSIF, PISANTY & STAUB**
**Modiano & Associati Via Meravigli, 16 16 16**
**I-20123 Milano(IT)**

(54) Connecting arrangement.

㊸ A connecting arrangement (10) incorporating a luer connector device and locking ring (26) for the luer connector components of the device and, more particularly, a connecting arrangement for the liquid transfusion between first and second tubing elements which is especially intended for explication in an intravenous administration apparatus, and wherein the arrangement facilitates the locking interengagement and simple separation of the interconnected luer components. The locking ring (26) incorporates a plurality of axially rearwardly extending flexures or tines (32) forming a sleeve-like structure which are adapted to resiliently engage into a reduced diameter portion (44) of the tubular surface about the circumference of the male luer component (24) when the locking ring (26) is moved into its operative position for engaging the female luer component (40). When it is intended to disengage the male and female luer components (24, 40), the flexures (32) on the locking ring (26) utilize the mechanical advantage provided for by rotation of the locking ring in the opposite direction of rotation to exert an axial biasing force against a shoulder (46) on the male luer component (24) at the rearward end of the reduced diameter portion (44) so as to separate the latter from engagement with the female luer component (40).

FIG. 3

# CONNECTING ARRANGEMENT

## BACKGROUND OF THE INVENTION

### 1. Field of the Invention

The present invention relates to a connecting arrangement incorporating a luer connector device and locking ring for the luer connector components of the device and, more particularly, pertains to a connecting arrangement for the liquid transfusion between first and second tubing elements which is especially intended for explication in an intravenous administration apparatus, and wherein the arrangement facilitates the locking interengagement and simple separation of the interconnected luer components.

The utilization of intravenous administration apparatus frequently entails the incorporation of luer connectors for the interconnection of fluid flow tubing or in conjunction with Y-connectors for such apparatus wherein a medicament is infused through the intermediary of a hypodermic needle into a parenteral fluid being administered through a catheter in a patient.

Generally, luer connector devices consist of cooperating male and female luer components which are maintained in an axially interengaged position during use through the employment of a so-called lock nut or locking ring. Ordinarily, a locking ring of that type incorporates internal screw threads and an inwardly depending shoulder or flange structure, the latter of which cooperates with a radially outwardly extending flange on the male luer component, whereas the internal screw threads engage complementary screw threads on the female luer components so as to be able to draw the luer components together and maintain them in a fluid-tight locked position. However, when it is intended to axially disengage and thereafter separate the male luer connector component from the complementary female luer component, this procedure usually necessitates imparting rotation to the lock nut and one of the luer components relative to the other, and/or the applying of an axial separating force thereto, such as through the physical application of a wrench-like tool, for instance, a hemistat. This frequently requires the exertion of large axial forces and the concommitant relative rotation between the luer components which may cause a patient connected to the system employing these components to be subjected to untenable degrees of discomfort.

### 2. Discussion of the Prior Art

In essence, Cross U. S. Patent 4,266,815 discloses a luer connector device for an intravenous administration apparatus in which a male luer component engages into a female luer component and is then fastened thereto through the intermediary of a locking ring. The locking ring incorporates internal screw threads engaging an upstanding flange member at one end of the female luer component forming an external screw thread whereas an inwardly depending annular shoulder on the locking ring contacts a shoulder or protuberance at the end of a recessed diameter portion of the male luer component so as to, in response to rotation of the ring, cause the luer components to be axially biased together into a locked position. However, in order to cause the disengagement between the male and female luer components, the screw threads of the locking ring must be disengaged from the flange on the female luer, and thereafter physical exertion applied between the luer connector in order to axially separate the male and female luer components. The foregoing is a cumbersome procedure which necessitates the expenditure of a considerable amount of separating force and relative rotation between the components, frequently causing discomfort to a patient due to extensive movement which has to be imparted to the entire intravenous administration apparatus.

Similarly, Ruschke U. S. Patent 4,452,473 discloses a luer lock connector system with interengaging male and female luer components, and a locking ring which includes internal screw threads engaging a flange on the female connector, and with a shoulder on the locking ring engaging an upstanding flange on the male connector so as to exially bias the two connecting parts into locking engagement upon rotation of the locking ring. In order to separate the male and female luer components, the locking ring must be rotated in an opposite direction of rotation, and thereafter axial force applied to the male and female luer components in order to cause them to disengage. This is also a cumbersome procedure somewhat analogous to that of the above described Cross patent and is subject to the same limitations and drawbacks.

Similar types of luer connecting devices are disclosed; for example, in Igari, et al. U. S. Patent 4,588,402; Muetterties, et al. U. S. Patent 4,296,949 and Gilson, et al. U. S. Patent 4,369,781, all of which disclose luer connectors incorporating lock nuts or locking rings engagable with screw thread portions on one or the other of the male or female luer components to draw these together into a locked condition. However, none of these structures enable the simple separation of the interengaged luer components in response to the rotation of the locking ring without the need for exerting external exial force or imparting relative rotation to the luer components.

## SUMMARY OF THE INVENTION

In order to obviate or ameliorate the limitations and drawbacks which are encountered in currently employed locking devices and connectors for fluid-flow tubing, such as in intravenous administration sets, employing interengaging male and female luer components, and to facilitate the simple separation of these components without the need for exerting extreme axial forces and in the absence of having to impart any relative rotation between the male and female luers, the present invention contemplates the utilization of a novel locking ring for the male and female luer connector components which locking ring may be maintained in an inoperative or standby position on a tubular member, preferably on the discharge tubing section of a Y-connector for an intravenous administration set, by grippingly engaging the surface thereof and wherein the fluid discharge end of the Y-connector includes a tubing segment having a male luer attached thereto which is adapted to matingly engage either a female luer or any other fluid-flow connecting device, such as leads to tubing for a catheter which is inserted into a patient. When the male luer component is intended to be engaged in fluid-flow connection into a complementary female luer component, then the locking ring, which may be referred to as a snap nut, is manually slid out of its inoperative or standby surface-gripping position on the tubular member and into an operative position for engagement with a flange provided on the female luer component in a manner analagous to that described with regard to the prior art devices.

The locking ring, in one embodiment thereof, incorporates a plurality of axially rearwardly extending flexures or tines forming a sleeve-like structure, which grippingly engage the surface of the discharge tubing section when the locking ring is in the operative position, and which are adapted to resiliently engage into a reduced diameter portion of the tubular surface about the circumference of the male luer component when the locking ring is advanced into its operative position for engaging the female luer component. When it is intended to disengage the male and female luer components, the flexures on the locking ring, which are radially resiliently flexible but axially substantially rigid, utilize the mechanical advantage provided for by rotation of the locking ring in the opposite or disengaging direction of rotation to exert an axial biasing force against a shoulder on the male luer component at the rearward end of the reduced diameter portion so as to separate the latter from engagement with the female luer component. Through this implementing of a simple rotational movement of the locking ring, it is possible that in the operative position thereof, there are interengaged and latched the male and female luer components in a manner similar to the prior art, while in addition thereto, upon the imparting of a counterrotation to the locking ring, there is produced an axial biasing force which separates the interengaged male and female luer components. This avoids the necessity for imparting large rotational forces and/or axial exertions on the interengaged components or having to impart relative rotation therebetween in order to disengage the luer lock. Consequently, the entire separating procedure becomes relatively simple and rapidly implementable, particularly when it is also desired to maintain the locking ring in its so-called inoperative or "stand-by" position distant from the luer lock when not employed in conjunction with the connection of the male luer discharge end of the tubular member to a female luer connector component.

Pursuant to another embodiment of the invention, instead of on the locking ring, flexures may be provided on the external surface of the male luer connector component, which are traversed by the locking ring when the latter is moved into its operative position for locking the male to the female luer component, and wherein the locking ring is contacted by the free end of the flexures during reverse rotation thereof so as to employ the mechanical advantage of such rotational movement to exert an axial biasing force against the male luer component which will separate the latter from the female luer component. The foregoing provides for an extremely simple luer connector design both as to construction and operation thereof, and eliminates the necessity of having to impart excessive manual separating forces to the interengaged luer components or having to utilize external separating tools, such as hemistats, wrenches or the like.

Accordingly, it is an object of the present invention to provide a connector arrangement for male and female luer lock components which incorporates a novel locking ring having structure facilitating the ready separation between the interengaged luer lock components through the imposition of an axial force applied to one of the luer components responsive to the mechanical advantage derived by rotation of the locking ring in a counter or unlocking direction.

## BRIEF DESCRIPTION OF THE DRAWINGS

Reference may now be had to the following detailed description of preferred embodiments of the connecting arrangement pursuant to the invention, taken in conjunction with the accompanying drawings; in which:

Figures 1 through 7 illustrate, in longitudinal section views, the inventive connecting arrangement employed on a Y-connector for an intravenous administration set shown in various operative positions of the luer locking device and of the locking ring pursuant to the invention which is associated therewith;

Figure 8 illustrates a longitudinal sectional view, on an enlarged scale, showing the locking ring on the male luer lock component; and

Figure 9 illustrates a modified embodiment of the inventive luer connecting arrangement.

DETAILED DESCIRPTION

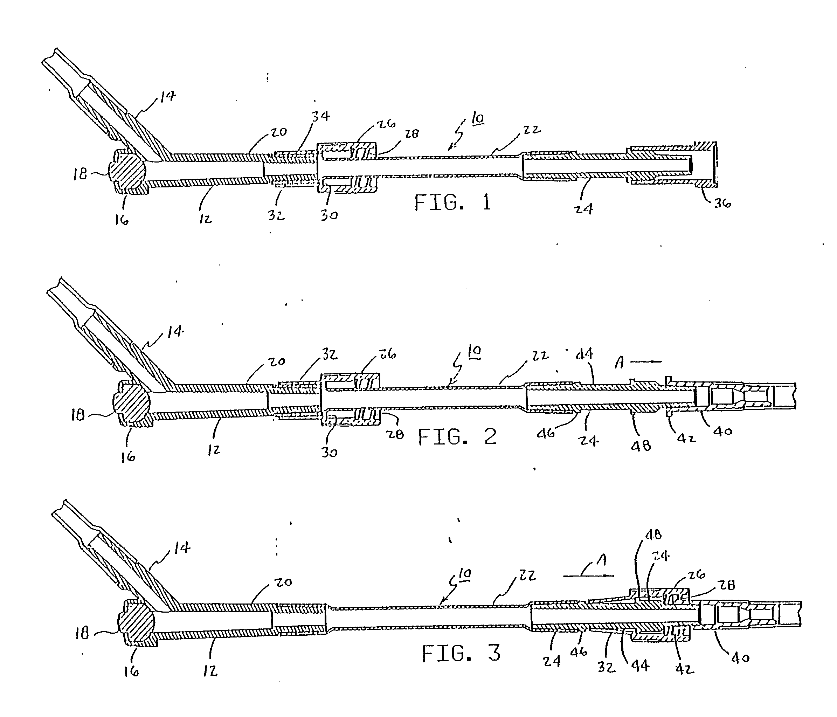

Referring now in more specific detail to the drawings, and particularly Fig. 1, there is illustrated a longitudinal sectional view through a connecting arrangement 10 which may be employed, for example, in conjunction with an intravenous administration set (not shown). For instance, a Y-connector 12 for use with such an IV administration set includes a tubular inlet arm 14 for the admission of a parenteral fluid, inlet arm 16 for the infusion of a medicament into the parenteral fluid, such as by the insertion of a hypodermic needle through a resealable puncturable member 18, and whereby the mixture of the parenteral fluid and the medicament introduced therein are advanced through the tubular arm member 20 of the Y-connector towards the discharge end thereof into an extension tubing 22, at other end of which there is featured a male luer component 24. Positioned on the tubular member 20 at the discharge end of the Y-connector 12 is a snap nut or locking ring 26 in an essentially inoperative or standby position thereof.

The locking ring 26 incorporates internal screw threads 28, and an inwardly extending flange or shoulder 30. Extending axially rearwardly from shoulder 30 are a plurality of resilient flexures or finger-like tines 32 essentially forming a sleeve about the circumference thereof, the flexures being of an essentially radially flexible but axially rigid nature, and which are in resiliently gripping engagement with a recessed circumferential surface 34 of the end of tubular arm member 20 of the Y-connector so as to maintain the locking ring 26 thereon when the latter is not in use.

When it is intended to remove a filter or hood element 36 which is mounted on the male luer component 24 so as to connect the male luer component 24 with a complementary female luer component 40, as shown in Fig. 2, whereby the latter may be connected to tubing leading to a catheter in a patient (not shown), then the tapered leading end of the male luer component 24 is slid into surface contacting engagement with the interior of the female luer component 40, as is known in the art.

The end of the female luer component 40 facing the male component 24 includes an upstanding flange portion 42, which may be either of an oval configuration, or merely include a narrow encompassing flange with two radially projecting diametrically opposite protrusions which are adapted to be engaged by the internal screw threads 28 on the locking ring 26 when the latter is moved into its operative position. In order to secure the female luer component 40 to the male luer component 24, as shown in Fig. 3, the locking ring 26 is advanced forwardly in the direction of arrow A from its inoperative position on tubular arm member 20 as shown in Figs. 1 and 2, by sliding the locking ring 26 and the flexures 32 thereon over the extension tubing 22 and the male luer component 24, until the flexures 32 resiliently engage into surface contact with a reduced diameter portion 44 on the male luer component 24 extending between a first rear shoulder 46 allowing the locking ring to pass thereover, and a larger diameter shoulder 48 which is adapted to be contacted by the inward shoulder 30 on the luer locking ring 26. When the ring 26 is then rotated, the internal screw threads 28 engage the upstanding portions of the flange 42 on the female luer component 40, which form an external screw thread, thereby drawing the female luer into tighter surface engagement over the tapered leading end of the male luer component 24, and with the shoulder 30 on the locking ring contacting the shoulder 48 to lock the luer connector components together.

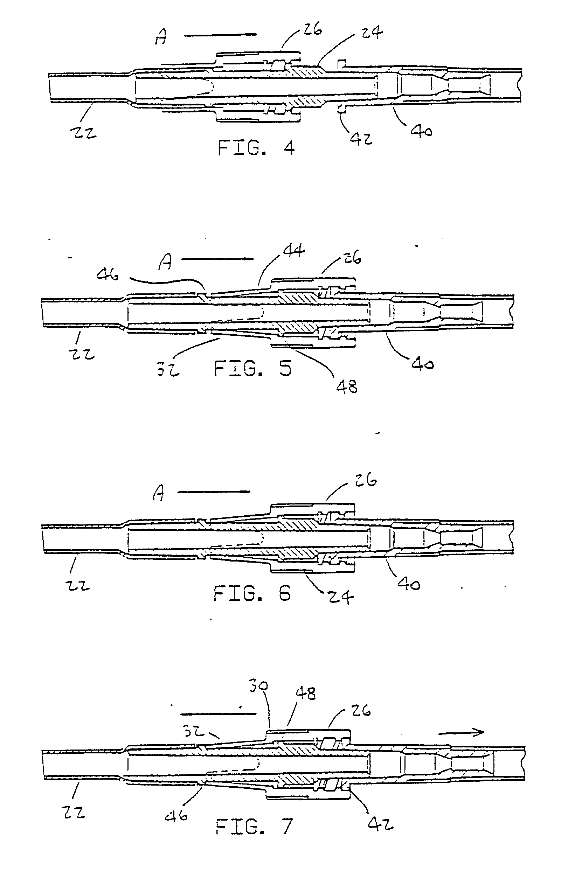

Referring in greater particularity to Figs. 4 through 7 of the drawings, Fig. 4 illustrates the advancing of the locking ring 26 from its standby or inoperative position shown in Fig. 1 towards the operative position shown in Fig. 3, shown in an intermediate stage prior to the internal screw threads 28 thereof threadingly engaging the flange 42 on the female luer connector component 40. In this instance, the flexures 32 of the locking ring 26 have resiliently expanded so as to enable the latter to pass over the shoulder 46 on the male luer component 24, and, as shown in Fig. 5, to bias radially inwardly so as to resiliently contact the reduced diameter surface 44 on the male luer component 24. Thereafter, upon imparting locking rotation to the locking ring 26, preferably in a clockwise direction as is common to such rotatable locking devices, the internal screw threads 28 of the locking ring 26 threadedly engage the flange or upstanding components 42 on the female luer component 40, and in view of the biasing action of the internal shoulder 30 of locking ring 26 acting against the shoulder 48 on the male luer component 24, draw the female luer component 40 into fluid tight surface contacting engagement with the male luer component 24, as shown in Fig. 6.

In order to produce a desired disengagement between the locked luer components 24 and 40, and without having to resort to the use of any external wrenching tools, such as hemistats, or to the application of excessive manual separating forces, it is merely necessary to rotate the locking ring 26 in an opposite direction, such as counterclockwise, which will displace the locking ring 26 backwards relative to flange 42 on the female luer component and cause the flexures 32 to have their rearwardly facing ends contacting against the shoulder 46 on the male luer component 24. Consequently, the internal screw threads 28 of the locking ring 26 are employed to derive a mechanical advantage for imparting an axial biasing force against the male luer component 24 to thereby axially push apart the male and female luer components in response to counterrotation of the locking ring. This is in clear contrast with conventional luer locks, in which the lock nut or locking ring is frequently employed to rotate the engaged luer components relative to each other so as to break these luer connections, and necessitates a greater amount of manual torque to be applied than the axial biasing force imparted by the mechanical advantage obtained by the locking ring screw threads and flexures pursuant to the invention.

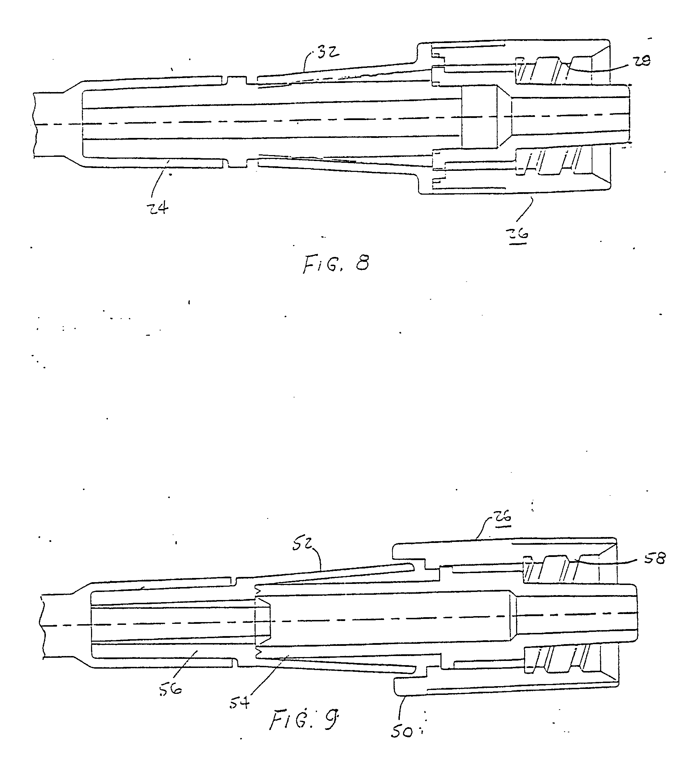

The locking ring 26 as disclosed in more specific detail in Fig. 8, has a plurality of such flexures 32 attached to or integrally molded with the body portion of the locking ring so as to extend circumferentially in order to form a sleeve-like structure constituted of a plurality of the axially-extending flexures in the shape of finger-like tines. The locking ring 26 may be constructed of a suitable polycarbonate plastic material, or any other comparable moldable plastic imparting the required mechanical strength and rigidity thereto.

Alternatively, as shown in the embodiment of Fig. 9 of the drawings, instead of providing flexures 32 on the locking ring 26, the latter may be primarily a standard type of locking ring 50 as presently employed in the art, and radially outwardly diverging flexures or tines 52 may be provided on the periphery of the male luer connector 54, possibly by being a portion of a ring-shaped attachment 56. Consequently, when the locking ring 50 is moved from an inoperative or standby position to an operative position, as in Figs. 1 to 3, the flexures 52 are temporarily depressed radially inwardly to permit passage thereover of the locking ring in order to enable the latter to have the internal screw threads 58 thereof contact a flange on a female luer component (not shown) and locking the latter against the male luer component. Thereafter, when it is desired to separate the male and female luer components, upon reverse rotation being imparted to the locking ring 50, the latter will now contact the ends of the radially diverging flexures 52 on the outer surface of the male luer component, and the mechanical advantage imparted by the screw threads of the locking ring are employed to exert an axial biasing force against the ends of the flexures tending to axially separate the male luer component from the interengaged female luer component. Again, as in the prior embodiment, this does not necessitate the utilization of any tools or excessive force for separating the luer connected components and, moreover, does not require any relative rotation between the luer connector components in order to separate the latter, thereby reducing any possible discomfort to a patient to an absolute minimum.

While there has been shown and described what are considered to be preferred embodiments of the invention, it will of course be understood that various modifications and changes in form or detail could readily be made without departing from the spirit of the invention. It is therefore intended that the invention be not limited to the exact form than the whole of the invention herein disclosed as hereinafter claimed.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A connecting arrangement for liquid transfusion between a first tubing device having a male luer connector at a discharge end thereof; and a second tubing device having a female luer connector at an inlet end, said female luer connector being engageable by said male luer connector so as to form a liquid-tight flow connection therebetween; and locking ring means being mounted on said first tubing device for axially slidable displacement therealong from an inoperative position on said first tubing device and an operative position on said male luer connector, said locking ring means including internal screw threads engageable with radially projecting shoulder means on said female luer connector upon rotation of said locking ring means in one direction so as to produce a locking engagement between said male and female luer components; and cooperating structure intermediate said first tubing device and said locking ring means responsive to rotation of said locking ring means in an opposite direction while in the operative position thereof to exert an axial biasing force against said male luer connector causing said male luer connector to axially disengage from said female luer connector.

2. A connecting arrangement as claimed in Claim 1, wherein said cooperating structure comprises an axially segmented tubular sleeve forming radially resilient flexures axially projecting from said locking ring means and extending coaxially rearwardly over said first tubing device, said radially resilient flexures being engageable into a reduced diameter surface portion on said male luer connector upon being slid thereover into the operative position thereof, and upon rotation of said locking ring means in said opposite direction, the rearward end surface of each of said flexures contacting against a shoulder at end of said reduced diameter portion on said male luer connector for imparting said axial biasing force thereto separating said male and female luer connectors from each other.

3. A connecting arrangement as claimed in Claim 2, wherein said flexure-forming sleeve is integrally formed with said locking ring means.

4. A connecting arrangement as claimed in Claim 2, wherein said locking ring means includes a radially inwardly depending shoulder; and a radially upstanding flange on said male luer connector at the forward end of said reduced diameter surface portion being contacted by said shoulder on said locking ring means for imparting a clamping force to said male and female luer connectors during rotation of said locking ring means in said one direction while in the operative position thereof.

5. A connecting arrangement as claimed in Claim 4, wherein said tubular sleeve includes a plurality of axially-extending circumferentially-spaced slits forming said flexures for imparting radially resilient properties thereto enabling said flexures to slide over said upstanding flange on said male luer connector and to engage in surface contact with the reduced diameter surface portion on said locking ring means when slidably displaced from the inoperative position towards the operative position along said first tubing device.

6. A connecting arrangement as claimed in Claim 1, wherein said radially projecting means on said female luer connector comprises an annular flange at the end of said female luer connector facing said male luer connector, and radially upstanding protuberances on said annular flange which are threadingly engageable with the internal screw threads on said locking ring means to form a locking interconnection between said male and female luer components and said locking ring means.

7. A connecting arrangement as claimed in Claim 2, wherein said tubular sleeve means comprises a plurality of axial flexures which are inherently rigid in an axial direction while being radially flexible.

8. A connecting arrangement as claimed in Claim 2, wherein said tubular sleeve is integrally formed with said locking ring means.

9. A connecting arrangement as claimed in Claim 1, wherein said male and female luer connector components and locking ring means are each constituted from a rigid plastic material.

10. A connecting arrangement as claimed in Claim 9, wherein at least said locking ring means is constituted from a polycarbonate plastic material.

11. A connecting arrangement as claimed in Claim 1, wherein said cooperating structure comprises a plurality of axially extending flexures encompassing at least a portion of the length of said male luer connector, said flexure each having one end thereof extending into a tubular sleeve connected between said male luer connector and said first tubing device, the free ends of said flexures extending towards the discharge end of said male luer connector component, each said flexure being axially rigid but radially flexible and diverging radially outwardly towards the free end thereof, said locking ring means being slidable over said flexure during displacement from the inoperative to the operative position by temporarily depressing said flexures radially inwardly during passage thereover and releasing said flexures upon reaching the operative position for engaging the female luer component, and said locking ring means including a radially inwardly extending annular flange contacting the free ends of said flexures in response to rotation of said locking ring means in said opposite direction so as to exert an axial biasing force against the ends of said flexures causing said male luer components to axially disengage from said female luer component.

12. A connecting arrangement as claimed in Claim 1, wherein said first tubing device comprises a component of an injection device for an intravenous administration set.

13. A connecting arrangement as claimed in Claim 1, wherein said second tubing device comprises an attachment for an intravenous catheter.

FIG. 1

FIG. 2

FIG. 3

FIG. 4

FIG. 5

FIG. 6

FIG. 7

FIG. 8

FIG. 9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US - A - 4 296 949 (MUETTERTIES et al.) * Column 1, lines 6-14; column 2, line 66 - column 3, line 29; column 4, line 23 - column 5, lines 5,44-48; fig. 1-4 * | 1,6,9, 10,12, 13 | A 61 M 39/00 |
| D,A | US - A - 4 452 473 (RUSCHKE) * Column 3, line 8 - column 4, line 33; fig. 6-8 * | 1,4,6, 9,10 | |
| D,A | US - A - 4 266 815 (CROSS) * Column 2, line 34 - column 4, line 15; fig. 1-5 * | 1,6,9, 10 | |
| A | US - A - 4 639 019 (MITTLEMAN) * Column 2, line 19 - column 3, line 16; fig. 1-3 * | 1,2,3, 6,9 | |

TECHNICAL FIELDS
SEARCHED (Int. Cl.5)

A 61 M   5/00
A 61 M  25/00
A 61 M  39/00
F 16 L  19/00
F 16 L  25/00
F 16 L  35/00

The present search report has been drawn up for all claims

| Place of search | Date of completion of the search | Examiner |
|---|---|---|
| VIENNA | 18-10-1990 | VELINSKY-HUBER |